# EUROPEAN PATENT SPECIFICATION

(11) **EP 3 102 276 B1**
(45) Date of publication and mention of the grant of the patent: **04.04.2018**
(21) Application number: 15708298.3
(22) Date of filing: 28.01.2015
(51) Int. Cl.: A61M 37/00, A61N 5/00

(54) **APPARATUS FOR THE TRANSDERMAL ADMINISTRATION OF PRODUCTS, FOR EXAMPLE OF PHYTOTHERAPY PRODUCTS OR THE LIKE**
VORRICHTUNG ZUR TRANSDERMALEN VERABREICHUNG VON PRODUKTEN, ZUM BEISPIEL PHYTOTHERAPIE- ODER ÄHNLICHEN PRODUKTEN
APPAREIL POUR L'ADMINISTRATION TRANSDERMIQUE DE PRODUITS, PAR EXEMPLE DE PRODUITS DE PHYTOTHÉRAPIE OU ANALOGUES

(30) Priority: 06.02.2014 IT MI20140170
(43) Date of publication of application: 14.12.2016
(73) Proprietor: Laserthru S.r.l., 20124 Milano (IT)
(72) Inventor: LANZETTA, Marco, I-20900 Monza (MB) (IT)
(74) Representative: Ripamonti, Enrico
(86) International application number: PCT/IB2015/050639
(87) International publication number: WO 2015/118427

(56) References cited:
- EP-A1- 1 752 190
- WO-A1-98/33444
- DE-C1- 3 905 517
- US-A- 5 999 847

## Description

The present invention refers to an apparatus for the transdermal administration of products, for example of phytotherapy products or the like.

A device like that described in the patent application EP 1752190 is known. Such medical machine is used for a purpose similar to that of the present apparatus (but limited to drugs), and has disadvantageous features with respect to the present invention. Indeed, it provides for the use of a frozen dispenser which is abutted against the skin during treatment, creating an unpleasant and painful sensation for the patient. This considerable discomfort for the patient can give potential problems due to skin lesions from prolonged cold ischemia, above all in elderly patients, children, carriers of skin diseases or those with cold skin intolerance.

In addition, it is of course necessary to always have a freezer available for maintaining the products -which must be prepared ahead of time with respect to the treatment to be executed - in a constant frozen state.

This involves, first of all, more difficult technical operations and secondly, in case of sudden power failure, the unfreezing of the products which must be suitably disposed of since they can no longer be used.

Finally, the known equipment does not allow the quantity of laser radiation supplied to the patient during a treatment to be known; additionally, it presents a non-uniformity in the application of the laser on the skin, thus making the treatment non-repeatable or non-programmable.

In addition, the action of the ultrasound head is not very effective since it does not transmit a sufficient power to the skin and to the product for facilitating the penetration of the latter in the skin.

An object of the present invention is therefore to obtain an innovative apparatus, without discomfort problems, with very advanced technology and which has unique features.

This and other objects are reached by an apparatus obtained according to the technical teachings of the attached claims.

One of the features that renders the present invention completely different from any other system or machinery is that fact that the product to be carried through the skin is enclosed in a semi-liquid aqueous gel and applied by means of an innovative applicator comparable to a dispenser of roll-on type.

The advantages of the invention are clear from the description of a preferred but non-exclusive embodiment of the apparatus, illustrated as a nonlimiting example in the attached drawings, in which:

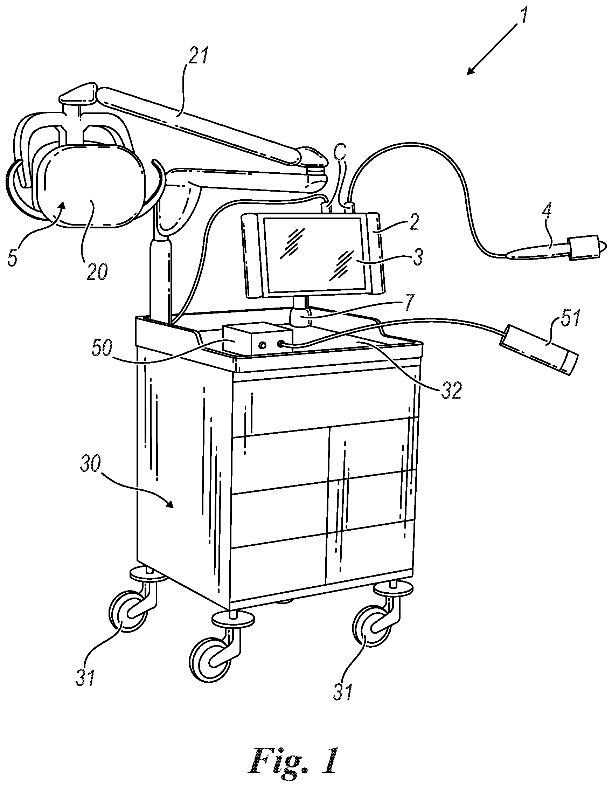

figure 1 is a simplified perspective view of the apparatus according to the present invention;
figure 2 is a perspective view of a handpiece of the apparatus of figure 1;
figure 3 is a simplified cross-section view of the handpiece of figure 2; and
figure 4 is a simplified and schematic perspective view of a detail of the apparatus of figure 1.

With reference to the abovementioned figures, an apparatus, as claimed in claim 1, is shown for the transdermal administration of products indicated overall with the reference number 1.

The apparatus 1 comprises a control unit 2 constituted by a metallic chassis provided with a front panel with a color LCD 3 screen, with 10" touchscreen that acts as user interface.

In order to facilitate its subsequent medical certification (as "low voltage" device), the control unit is power supplied with an external power supply of portable PC type. This ensures the universal nature of the power supply source (90-260V) and a high level of electrical safety, even if the required isolation characteristics are less stringent than, for example, an electrocardiograph or electromyograph which must be of "BF" class since it has electrical connections towards the patient.

Within a chassis, there is an actual computer of industrial type; this is capable of sustaining mechanical and thermal stresses greater than that which it will in reality sustain. Advantageously, it lacks a cooling fan and is thus completely silent, as well as clean since it does not suction nor accumulate dust. The mass memory is preferably a solid state disc, which renders the device completely unaffected by vibrations and also suitable for a movable/transportable use.

In addition, the chassis also comprises the circuit boards suitably designed and manufactured for controlling the handpiece 4 and a scanner 5, which will be described hereinbelow.

The use of a true (industrial) PC architecture involves numerous advantages, including, for example, the availability of standard expansion ports, for interfacing with future external peripheral devices (e.g. printer) or the insertion in a network (e.g. a LAN network of devices with centralized database); the expansion ports (USB, Ethernet, video output) are already available on the board and accessible by means of an expansion/maintenance bay that is normally closed. There is also the possibility for expansion by means of the installation of standard peripheral devices such as a WiFi network card or a RFID transponder. The latter is a module, whose space is already arranged behind the front panel, which can read and recognize the contactless smartcards (identical to modern hotel keys) used as unique identification of the patient and operator. These cards can be used for identifying and logging the sessions made by each operator, for the automatic setting of the treatment parameters as a function of the recognized patient, etc.

Two identical connectors C are installed on the upper part of the equipment. Connected therewith are the handpiece 4 and the adjustable head of the scanner 5. The electrical connections are preferably obtained in a manner so as to allow the non-preferential use of one or the other.

The equipment further comprises a 45° table stand support. The connection for the stand has VESA standard (the same found on the rear of televisions) allowing, if desired, the wall installation of the console by using standard, inexpensive adjustable TV arms.

The man-machine interface software allows the complete control of the machine through the touchscreen; with respect to the use of keys, this ensures greater ease of cleaning.

The controls are quick and intuitive, with an interface as simple and linear as possible and made for "finger use" in order to be easily controllable: the controls are all positioned on a single main screen in a manner so as to be immediately accessible without having to change page.

The software is multilingual, and among the various languages the following are present: English, Italian, Spanish, French and Swedish.

The handpiece 4 is the unit that can be gripped for applying the gel, connected to the console by means of a cable that can be disconnected.

The handpiece 4 uses piston cartridges, to which a roll-on head has been applied on the cap.

In substance, the handpiece comprises a support structure 10, preferably metallic and externally knurled for a secure grip.

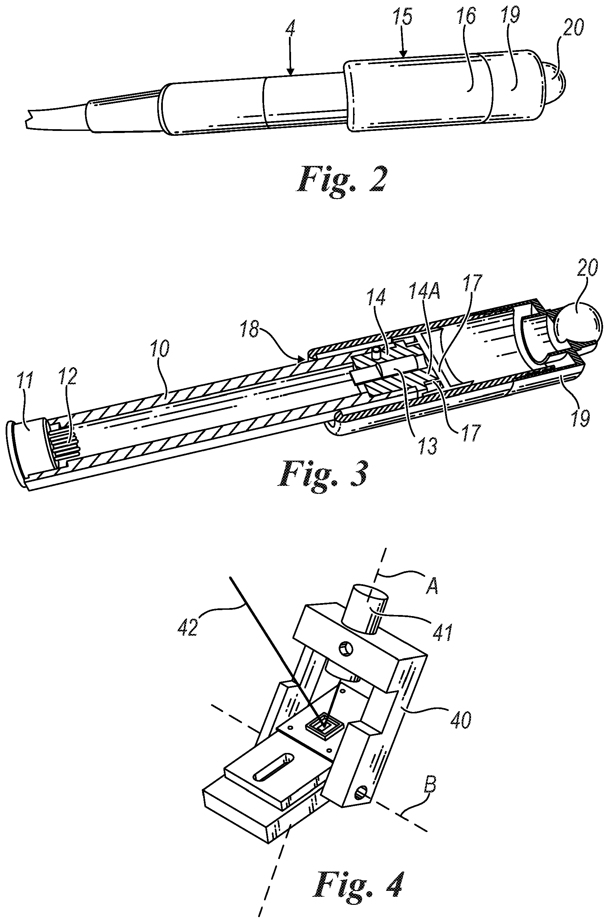

At one end of the metallic structure, a connector 11 is fixed, as is seen in the section of figure 3; such connector 11 is provided with electrical terminals 12 to which wires (not shown) are connected that are connected to a laser source fixed to a support 14 fixed to the opposite end of the structure 10.

The laser source is positioned in a manner so as to emit a laser beam that exits from one end of the structure 10.

The structure 10 has an external diameter such to allow its removable coupling with a cartridge 15 in which the product suitable to be distributed on the skin of the patient is contained.

In substance, the external surface of the handpiece constitutes a removable means for fixing the same to the cartridge 15.

The product can for example be an active medical ingredient of any type, a conventional drug, a phytotherapy product or natural supplement, a collagen product based on collagen or hyaluronic acid, a chemotherapeutic substance or a cosmetic product, or any other compound that can be introduced through the skin.

The cartridge 15 has an external casing 16 with tubular shape. Such casing has a hole 18 on the bottom that allows the casing itself to fit, with slight friction, on the structure 10 of the handpiece. The support 14 then has a protuberance that is inserted, it too with slight friction, in a suitable seat 17A obtained in a slidable piston 17 preferably sealingly slidable within the casing 16.

Such casing on the side opposite the hole 18 has a thread (or other suitable means) for coupling a cap-dispenser 19. The cap is provided with a shaped portion adapted to rotatably retain a small roll-on ball 20, a part of which being in contact during use with the product inside the casing 16.

The small ball 20, along with the piston 17, are made of a transparent or semi-transparent (translucent) material thus allowing the laser light to traverse both components in order to operate on the product applied on the patient skin.

Unlike the conventional handpieces in which the product intended for application must be frozen in order to remain in position, by means of a small ball-shaped head, use is allowed of a semi-liquid product or gel, with a very easy and quick application, without waste and without dirtying.

The handpiece has a laser module or source 13 mounted thereon whose power can if desired be adjusted, "reducing" the maximum power thereof by a control of the software, between 0% and 100%. This is managed by the suitably designed control board, which drives the laser in PWM (Pulse Width Modulation), i.e. with an on-off signals that gives and removes power supply hundreds of times per second, varying the duration of the activation phase.

The maximum power of the laser is advantageously 5mW.

The scanner 5 is the part of the equipment that illuminates the skin with the laser radiation after the application phase. It is composed of a compact and light head 20 mounted on a support 21 that can be adjustably articulated as desired (goose-neck). This is preferably fixed and supported on a furniture piece 30 provided with wheels 31 for a quick movement. The furniture piece 30 further comprises a work surface 32, to which the stand is also fixed.

From the support base 21, an electric cable departs that is connected to the scanner and which terminates in a connector that can be coupled with a connector C of the control unit 2.

The head of the scanner contains a mechanical structure 40, clearly visible in figure 4, which supports a laser emitter 41, it too preferably with 5mW power; the beam 42 is directed towards the surface of a MEMS device that deflects it in the desired direction. The MEMS is a so-called micro-electromechanical device (Micro Electro-Mechanical System), and is composed of a very small, electrically-controlled actuator comprising a mirror with about 1 mm diameter mounted on hinges that allow it to rotate independently on two axes (A and B). The first axis is responsible for the horizontal scanning of the laser beam, the second axis is responsible for the lateral scanning.

By suitably controlling the device, the mirror deflects the laser beam in any desired direction (of course within the operating limits of the hinges).

During operation, the two axes of the mirror continuously oscillate with two different frequencies; the so-called "fast" axis with a frequency of about 21 kHz (21,000 times/s) and the other ("slow axis") with a frequency of 1500 Hz. The result is that the laser point travels approximately sinusoidal trajectories being slightly moved with each cycle.

While the slow axis B generates an oscillation along one axis (the horizontal axis), the fast axis A executes different oscillations along the other axis (the lateral axis). Since the two oscillation frequencies are not exact multiples, the starting point is slightly different at the subsequent oscillation. The result is that the continuous oscillation of the two axes fully covers the surface of a rectangle. By varying the amplitude of the oscillation on the two axes, the size of the sides of the rectangular are adjusted.

The deflection speed is so high that the eye does not perceive the laser point and its sinusoidal trajectories, but rather sees the area of the rectangle approximately illuminated in a uniform manner.

The advantages in the use of this solution are the absolute silence of the device, the reliability (the MEMS are not subject to wear), the mechanical simplicity, to the benefit of maintenance and reliability, the limited bulk and weight, the lighting uniformity and reduced power waste, since all the energy is emitted within the delimited area, the greater versatility and field of regulation, even creating non-rectangular forms by modifying the amplitude of the oscillation along the axis.

As stated above, the horizontal scanning (slow axis B) of the laser beam is obtained electromechanically by driving a servomechanism on which the mirror is mounted.

By using a constant angular rotation speed for the slow axis, it follows that, while the beam below the vertical of the scanner head, the linear speed of the beam is lower, while it increases as it approaches the edges. When the rotation speed is constant (varies linearly), the linear speed on the skin (i.e. on the area of the rectangle) will be higher the more it is removed from the vertical. It follows that the exposure to the laser will be fully non-uniform and the peripheral zones will be less exposed, especially for extensive treated zones (very large area of the rectangle, like when the back of a patient must be treated or if the head of the scanner is relatively close to the skin).

Advantageously, in addition to this scan type, a second type of "compensated" scanning is available, in which the rotation speed along the slow axis B (or horizontal axis) is varied in a manner proportional to the arc tangent of the angle of the beam with respect to the vertical, i.e. more quickly at the vertical, and more slowly at the edges. This compensation ensures that the linear scanning speed is constant, and that the entire area of the rectangle (and hence of the treated skin) receives the same quantity of laser radiation.

In the provided embodiment, the device also provides for an ultrasound 50 that comprises an ultrasound head 51.

As is known, each ultrasound head has a characteristic bell-shaped resonance curve. Such curve represents the power of the ultrasound signal as a function of the excitation frequency.

Normally, the curve is very narrow and has a maximum at the nominal resonance frequency. Even moving only a few Hz higher or lower with respect to the nominal resonance frequency will cause the output power (and hence the "effectiveness" of the ultrasound vibration) to drop drastically; it follows that the head must be driven at a frequency as identical as possible to that of nominal resonance.

If, due to the pressure of the ultrasound head on the skin, the mechanical characteristics vary even slightly, the resonance curve can be altered and the resonance frequency can be moved with respect to the nominal frequency. Hence, by continuing to drive at such fixed frequency, the output power results considerably reduced.

In addition, due to the inevitable manufacturing tolerances, in machine production phase it would be necessary to calibrate the exact oscillation frequency for each head, and possibly provide a periodic recalibration - with considerable service costs.

For this reason, in the device according to the present invention, the head is driven with a frequency "sweep", i.e. the oscillation frequency is continuously varied between a range of values close to that of nominal resonance. In this manner, there will always be a moment when the drive frequency is identical to the actual resonance frequency, and the emissivity is therefore maximum, even after alterations of the characteristic curve due to aging, pressure of the head on the skin, or other incontrollable factors.

In addition, this avoids the costs of a periodic calibration of the machine.

Clearly, the disadvantage of this technique is that there is a time when the head is driven at a sub-maximal power, and hence the average power during a sweep will be less than the peak power; this can be easily compensated for by increasing the drive power or selecting a head with greater power. On the contrary, with a driving at fixed frequency, the output power would be less controlled so that it could be constantly close to the peak power (if the drive frequency is very similar to the resonance frequency), or it could be constantly different from the peak power.

The frequency sweep is advantageously executed with a scan time of about 3-4s, in a range of frequencies with amplitude of about 5% with respect to the nominal resonance frequency (around 40 kHz) and centered around such frequency; such amplitude is reasonably greater than the manufacturing tolerances (2-3%) and the alterations of the nominal frequency that are expected, in a manner so as to limit the time in which the head is driven at sub-maximal power.

From that described above, the operation of the finding is evident for the man skilled in the art, and in particular is the following.

The apparatus provides for the use of an ultrasound head 51 in addition to the two lasers 4 and 5. In substance, the skin of a patient is treated first with ultrasound by means of the head 51; subsequently, the handpiece 4 is used which irradiates the skin with a laser beam and simultaneously distributes the product contained in the casing 16 on such skin, by means of the small ball 20. The use of such handpiece renders the application quick, precise, without waste and without discomfort for the patient.

Finally, the skin on which the product has been applied is further irradiated with a laser by means of the scanning head 5.

Ample literature demonstrates that the sound waves emitted by a pretreatment of the skin with ultrasound facilitate the transdermal passage of compounds by opening the intercellular channels.

Advantageously associated with the scanning laser 5 is a detector of energy emitted on the skin, which always ensures a constant control of the zone that it treated during the treatment session. Such detector is for example a meter that, based on the area swept by the beam, on the exposure time and on the distance of the source from the skin, determines the theoretical quantity absorbed by the skin. Such function is implemented by the control unit and the result of such calculation is displayed on the screen 3.

Such detector or meter of emitted energy is particularly useful in coupling with suitable means in order to be able to vary the power emitted by the lasers, by means of the control on the console.

The detector can also be a physical sensor rested on a portion of skin treated during the treatment, which detects the quantity of incident laser per unit of surface area.

In substance, an apparatus is described for the transdermal administration of a product in the fluid state, comprising a handpiece provided with a support structure within which a laser source is positioned in a manner such that the laser beam emitted by said laser source exits from a first end of said support structure. The handpiece comprises a cartridge positioned in proximity to said first end and containing said product in the fluid state, which is preferably in gel form. The cartridge is fixed to said support structure by means of removable fixing means in a manner such that the laser beam axially traverses the cartridge. It has a tubular external casing perforated on its bottom, and a piston sealingly slidable in said tubular casing; the casing, on the side opposite said bottom, has a ball-shaped applicator for distributing the contents of said cartridge. The ball and the piston are made of a transparent or semi-transparent material adapted to allow the light of said laser to cross them, thus also illuminating the product distributed on the skin.

The apparatus then comprises an ultrasound head and a further generator of a further laser beam coupled to means for moving the latter within a settable treatment area.

Preferably the ultrasound head has means adapted for exciting it with a frequency centered on the resonance frequency of the head and variable between ± 5% of said resonance frequency. For example, said means adapted to excite the head with a variable frequency cyclically vary the frequency in a time comprised between 3 and 4 seconds.

In a preferred configuration, said further means for generating the further beam comprise a laser emitter that projects its beam towards the surface of a mirror mounted on hinges, which allow the mirror to rotate around at least two axes; the rotation of the mirror around the two axes is controlled by actuators.

The means direct the further laser beam in a manner so as to irradiate each part of the treated area with a same quantity of laser radiation.

The actuator adapted to rotate the mirror around one of said two axes, which generates a horizontal scanning of the beam, oscillates with a variable speed with the arc tangent of the angle formed between the direction of the beam and the vertical passing through mirror.

Preferably means are present for measuring the quantity of radiation supplied to the skin by the further laser beam.

In a preferred embodiment, the measuring means determine the quantity of radiation based on the power of the laser, of the distance from the skin, on the swept area and on the exposure time.

In substance, a method is described for the transdermal administration of a product comprising the steps of: treating the part of skin intended to receive the product with an ultrasound head, following the treatment with ultrasound applying the product on the skin, simultaneously irradiating both the product and the skin with a laser beam, and after having applied the product on the skin irradiating the area treated with the product with a further laser beam.

Preferably the skin is treated with an ultrasound head excited with a variable frequency centered on the resonance frequency of the head itself and varying between ± 5% of said resonance frequency, in a time comprised between 3 and 4 seconds.

The product is applied by means of a handpiece provided with a support structure, within which a laser source is positioned in a manner such that the laser beam emitted by said laser source exits from a first end of said support structure, the handpiece further comprising a cartridge positioned in proximity to said first end and containing said product in the fluid state, preferably in gel form, removably fixed to said support structure by means of removable fixing means in a manner such that said laser beam axially traverses the cartridge, the cartridge having a tubular external casing open on its bottom and a piston sealingly slidable in said tubular casing; the casing, on the side opposite said bottom, having a ball-shaped applicator for distributing the contents of said cartridge, said ball and said piston being made of a transparent or semi-transparent material adapted to allow the light of said laser to cross them.

One variant provides that the beam is generated by means of a laser emitter that projects its beam towards the surface of a mirror mounted on hinges, which allow the mirror to rotate around at least two axes; the rotation of the mirror around the two axes is controlled by actuators.

Advantageously the further laser beam is moved in a manner so as to irradiate each part of the treated area with a same quantity of laser radiation. The movement of the beam occurs by means of the simultaneous movement of the mirror.

According to a preferred embodiment, the rotation speed of the mirror around one of said two axes which generates a horizontal scanning of the beam, is made to vary according to the arc tangent of the angle.

Advantageously, the quantity of radiation supplied to the skin by the further laser beam is then measured, preferably by means of a meter that determines the theoretical quantity thereof, being based on the power of the laser, of the distance from the skin, on the swept area and on the exposure time.

## Claims

1. Apparatus for the transdermal administration of a product in the fluid state comprising a handpiece (4) provided with a support structure (10) within which a laser source (13) is positioned in a manner such that the laser beam emitted by said laser source (13) exits from a first end of said support structure (10), the handpiece (4) further comprising a cartridge (15) positioned in proximity to said first end and containing said product and fixed to said support structure (10) by means of removable fixing means in a manner such that said laser beam axially traverses the cartridge (15), the cartridge having an tubular external casing (16) perforated (in 18) on its bottom, and a piston (17) sealingly slidable in said tubular casing (16), wherein that said product is in the fluid state, preferably in gel form, **characterized in that** the casing (16), on the side opposite said bottom, has a ball-shaped applicator (20) for distributing the contents of said cartridge (15), said ball (20) and said piston (17) being made of a transparent or semi-transparent material adapted to allow the light of said laser source (13) to cross them, thus also illuminating the product distributed on the skin.

2. Apparatus according to claim 1, **characterized in that** it comprises an ultrasound head (51) and a generator (41) of a further laser beam (42) coupled with means for moving the latter to the interior of a settable treatment area.

3. Apparatus according to the preceding claim, wherein the ultrasound head (51) has drive means adapted to vary the oscillation frequency in a range of frequency values close to the resonance frequency and with amplitude equal to ± 5% with respect to said resonance frequency.

4. Apparatus according to the preceding claim, wherein the frequency is cyclically varied in a time comprised between 3 and 4 seconds.

5. Apparatus according to one or more of the preceding claims, wherein said further means for generating the further laser beam (42) comprise a laser emitter (41) which projects its beam (42) towards the surface of a mirror mounted on hinges which allow it to rotate around at least two axes, the rotation of the mirror around the two axes being controlled by actuators.

6. Apparatus according to claim 5, wherein said means direct the further laser beam (42) in a manner so as to irradiate each part of the area treated with a same quantity of laser radiation.

7. Apparatus according to one or more of the preceding claims, wherein said actuator adapted to rotate the mirror around one of said two axes, which generates a horizontal scanning of the beam, oscillates with a variable speed with the arc tangent of the angle formed between the direction of the beam and the vertical passing through mirror.

8. Apparatus according to one or more of the preceding claims, wherein means are present for measuring the quantity of radiation supplied to the skin by the further laser beam (42).

9. Apparatus according to the preceding claim, wherein said measuring means determine the quantity of radiation based on the power of the laser, on the distance from the skin, on the swept area and on the exposure time.

## Patentansprüche

1. Vorrichtung zur transdermalen Verabreichung eines Produkts im flüssigen Zustand, umfassend ein Handstück (4) mit einer Stützstruktur (10), in der eine Laserquelle (13) angeordnet ist, so dass der von der genannten Laserquelle (13) emittierte Laserstrahl aus einem ersten Ende der genannten Stützstruktur (10) austritt, wobei das Handstück (4) eine Kartusche (15) weiter umfasst, die in der Nähe von dem genannten ersten Ende liegt und das genannte Produkt enthält und an der genannten Stützstruktur (10) durch abnehmbare Befestigungsmittel befestigt ist, so dass der genannte Laserstrahl die Kartusche (15) axial durchquert, wobei die Kartusche eine an dem Boden (in 18) gelöcherten äußeren röhrenförmigen Hülle (16) und einen in der genannten röhrenförmigen Hülle (16) dichtend verschiebbaren Kolben (17) hat, worin das genannte Produkt in flüssigem Zustand, vorzugsweise in Form eines Gels, ist, **dadurch gekennzeichnet, dass** die Hülle (16) an der dem genannten Boden gegenüberstehenden Seite einen kugelförmigen Applikator (20) zur Verteilung des Inhalts der genannten Kartusche (15) hat, wobei die genannte Kugel (20) und der genannte Kolben (17) aus einem transparenten oder halbtransparenten Stoff hergestellt werden, der sich eignet, die Licht der genannten Laserquelle (13) dadurch zu lassen, so dass das auf der Haut verteilte Produkt auch beleuchtet wird.

2. Vorrichtung nach Anspruch 1, **dadurch gekennzeichnet, dass** es einen Ultraschallkopf (51) und einen Generator (41) eines weiteren Laserstrahls (42), der mit Mitteln zur Bewegung davon bis zu einem einstellbaren Behandlungsbereich zugeordnet ist, umfasst.

3. Vorrichtung nach dem vorherigen Anspruch, worin der Ultraschallkopf (51) Antriebsmittel hat, die sich eignen, die Schwingungsfrequenz in einem Bereich von Frequenzwerten, die in der Nähe von Resonanzfrequenz liegen und die eine Amplitude von ± 5 % relativ zu der genannten Resonanzfrequenz haben, zu ändern.

4. Vorrichtung nach dem vorherigen Anspruch, worin die Frequenz in einer Zeit zwischen 3 und 4 Sekunden zyklisch geändert wird.

5. Vorrichtung nach einem oder mehreren der vorherigen Ansprüche, worin die genannten weiteren Mittel zur Erzeugung des weiteren Laserstrahls (42) einen Lasersender (41) umfassen, der den entsprechenden Strahl (42) gegen die Oberfläche eines an Scharnieren gelagerten Spiegels projiziert, wobei die Scharniere dem Spiegel ermöglichen, um mindestens zwei Achsen zu drehen, wobei die Drehung des Spiegels um die beiden Achsen durch Aktoren gesteuert wird.

6. Vorrichtung nach Anspruch 5, worin die genannten Mittel den weiteren Laserstrahl (42) derart richten, dass jede Portion des behandelten Bereichs mit derselben Menge von Laserstrahlung gestrahlt wird.

7. Vorrichtung nach einem oder mehreren der vorherigen Ansprüche, worin der genannte Aktor, der den Spiegel um eine der genannten zwei Achsen dreht und eine horizontale Abtastung des Strahls erzeugt, mit veränderlicher Geschwindigkeit schwingt, wobei die Bogentangente des zwischen der Richtung des Strahls und der Vertikale gebauten Winkels durch den Spiegel verläuft.

8. Vorrichtung nach einem oder mehreren der vorherigen Ansprüche, worin Mittel zur Messung der Menge von durch den weiteren Laserstrahl (42) der Haut gelieferten Strahlung vorhanden sind.

9. Vorrichtung nach dem vorherigen Anspruch, worin die genannten Messmittel die Menge von Strahlung anhand der Leistung des Lasers, des Abstands von der Haut, des abgetasteten Bereichs und der Belichtungszeit feststellen.

## Revendications

1. Appareil pour l'administration transdermique d'un produit à l'état fluide, comprenant une pièce à main (4) équipée d'une structure de support (10) à l'intérieur de laquelle une source laser (13) est positionnée de sorte que le faisceau laser émis par ladite source laser (13) sort d'une première extrémité de ladite structure de support (10), la pièce à main (4) comprenant aussi une cartouche (15) positionnée à proximité de ladite première extrémité et contenant ledit produit et fixée à ladite structure de support (10) par des moyens de fixation amovibles, de sorte que ledit faisceau laser traverse axialement la cartouche (15), la cartouche ayant une enveloppe tubulaire (16) extérieure perforée (en 18) sur son fond et un piston (17) coulissant de manière étanche dans ladite enveloppe tubulaire (16), où ledit produit est à l'état fluide, préférablement sous forme de gel, **caractérisé en ce que** l'enveloppe (16), sur le côté opposé audit fond, a un applicateur en forme de bille (20) pour distribuer le contenu de ladite cartouche (15), ladite bille (20) et ledit piston (17) étant réalisés en un matériau transparent ou semi-transparent adapté pour permettre à la lumière de ladite source laser (13) de les traverser, de cette manière éclairant aussi le produit distribué sur la peau.

2. Appareil selon la revendication 1, **caractérisé en ce qu'**il comprend une tête à ultrasons (51) et un générateur (41) d'un faisceau laser (42) supplémentaire couplé avec des moyens pour le déplacer à l'intérieur d'une zone de traitement réglable.

3. Appareil selon la revendication précédente, où la tête à ultrasons (51) a des moyens d'entraînement adaptés pour varier la fréquence d'oscillation dans un intervalle de valeurs de fréquence près de la fréquence de résonance et avec une amplitude égale à ± 5 % par rapport à ladite fréquence de résonance.

4. Appareil selon la revendication précédente, où la fréquence est cycliquement variée en un temps entre 3 et 4 secondes.

5. Appareil selon une ou plusieurs des revendications précédentes, où lesdits moyens supplémentaires pour générer le faisceau laser (42) supplémentaire comprennent un émetteur laser (41) qui projette son faisceau (42) vers la surface d'un miroir monté sur des charnières qui lui permettent de tourner autour d'au moins deux axes, la rotation du miroir autour des deux axes étant contrôlée par des actionneurs.

6. Appareil selon la revendication 5, où lesdits moyens dirigent le faisceau laser (42) supplémentaire de sorte à irradier chaque partie de la zone traitée par la même quantité de radiation laser.

7. Appareil selon une ou plusieurs des revendications précédentes, où ledit actionneur adapté pour tourner le miroir autour d'un desdits deux axes, ce qui génère un balayage horizontal du faisceau, oscille à une vitesse variable avec l'arc tangente de l'angle formé entre la direction du faisceau et la ligne verticale traversant le miroir.

8. Appareil selon une ou plusieurs des revendications précédentes, où des moyens sont présents pour mesurer la quantité de radiation fournie à la peau par le faisceau laser (42) supplémentaire.

9. Appareil selon la revendication précédente, où lesdits moyens de mesure déterminent la quantité de radiation sur la base de la puissance du laser, de la distance de la peau, de la zone balayée et du temps d'exposition.
